# EUROPEAN PATENT SPECIFICATION

(11) **EP 3 456 275 B1**
(45) Date of publication and mention of the grant of the patent: **15.04.2020**
(21) Application number: 17191758.6
(22) Date of filing: 19.09.2017
(51) Int. Cl.: A61B 17/22

(54) **CARBON DIOXIDE FOR TREATING PEYRONIE`S DISEASE**
KOHLENDIOXID ZUR BEHANDLUNG VON PEYRONIE-KRANKHEIT
DIOXYDE DE CARBON POUR LE TRAITEMENT DE LA MALADIE DE PEYRONIE

(43) Date of publication of application: 20.03.2019
(73) Proprietor: BMR Medical LLC, Marietta, GA 30067 (US)
(72) Inventor: MORGANSTERN, Steven, Sandy Springs, GA Georgia 30328 (US); BECERRA, Carlos, Atlanta, GA Georgia 30328 (US)
(74) Representative: Viering, Jentschura & Partner mbB Patent- und Rechtsanwälte

(56) References cited:
- RU-C1- 2 256 405
- US-A1- 2012 215 142
- US-A1- 2013 245 541
- US-A1- 2015 073 312
- US-A1- 2017 258 674

## Description

### BACKGROUND OF THE INVENTION

### 1. Field of the Invention

The present invention relates to treatment methods and, more specifically, to a method of treating Peyronie's disease.

### 2. Description of the Related Art

Peyronie's disease (also known as induratio penis plastic) is an acquired inflammatory condition of the penis associated with penile curvature. It is a connective tissue disorder involving the growth of fibrous plaques in the soft tissue of the penis. Scar tissue forms in the tunica albuginea, the thick sheath of tissue surrounding the corpora cavemosa causing pain, abnormal curvature, erectile dysfunction, indentation, loss of girth and shortening. The penile curvature of Peyronie's disease is caused by an inelastic scar, or plaque (which may include calcification), that shortens the involved aspect of the tunica albuginea of the corpora cavemosa during erection.

If left untreated, Peyronie's disease may cause fibrotic, nonexpansile thickening of relatively discrete areas of the corpora tunica, typically resulting in focal bend, pain or other functional or structural abnormalities of the erect penis. Surgery is one method of treating Peyronie's disease. Surgery has the disadvantage of being expensive and occasionally resulting in unwanted complications. Several medical treatments have been applied, but results so far have been limited. Surgical treatments have also been used to treat Peyronie's disease. Collagenase clostridium histolyticum (marketed as Xiaflex), an injectable drug, is the most common medical treatment of Peyronie's disease. It is believed that this works by breaking down the excess collagen in the penis that causes Peyronie's disease. This drug has limited success and it can be quite expensive. Known methods for treating Peyronie's disease through shockwave therapy are described in patent publications US 2012/0215142 A1 and RU 2256405 C1.

Therefore, there is a need for a reliable and inexpensive non-surgical treatment for Peyronie's disease.

### SUMMARY OF THE INVENTION

The disadvantages of the prior art are overcome by the present invention which, in one aspect, is a method of treating a patient having diffused plaque and a plaque mass associated with Peyronie's disease in a penile region, in which a battery of tests is performed to quantify an initial state of parameters associated with Peyronie's disease in the patient. Low intensity shock wave therapy is applied to the plaque mass in the penile region, thereby softening the plaque mass and disrupting any calcification in the plaque mass. Carbon dioxide is injected into the plaque mass. The battery of tests is repeated to quantify a current state of parameters associated with Peyronie's disease in the patient and the current state is compared to the initial state. The aforementioned treatment steps are repeated until the current state differs from the initial state by at least a predetermined amount.

In another aspect, the invention is a treatment method for a patient having a plaque mass associated with Peyronie's disease in a penile region, in which a battery of tests is performed to quantify an initial state of parameters associated with Peyronie's disease in the patient. Low intensity shock wave therapy is applied to a plaque mass in the penile region for about thirteen treatments over a period of seven to eight weeks including a two week break period of no low intensity shock wave treatment, thereby softening the plaque and disrupting calcification in the plaque mass. A plurality of doses of about 160 cc of carbon dioxide per dose is injected into the plaque mass for a total of 960 cc. A counter pulsation treatment is applied every five days during a period of seven weeks to the patient concurrently with the step of applying low intensity shock wave therapy to further disrupt calcification in the plaque mass. A therapeutically effective dose of Verapamil is injected into a dorsal area of the penile area after the step of applying low intensity shock wave therapy to the plaque mass. The battery of tests is repeated to quantify a current state of parameters associated with Peyronie's disease in the patient and comparing the current state to the initial state. The aforementioned steps are repeated until the current state differs from the initial state by at least a predetermined amount.

These and other aspects of the invention will become apparent from the following description of the preferred embodiments taken in conjunction with the following drawings. As would be obvious to one skilled in the art, many variations and modifications of the invention may be effected without departing from the spirit and scope of the novel concepts of the disclosure.

### BRIEF DESCRIPTION OF THE FIGURES OF THE DRAWINGS

**FIG. 1** is a schematic diagram showing treatment of a dorsal plaque.
**FIG. 2** is a flow chart showing a treatment protocol.

### DETAILED DESCRIPTION OF THE INVENTION

A preferred embodiment of the invention is now described in detail. Referring to the drawings, like numbers indicate like parts throughout the views. Unless otherwise specifically indicated in the disclosure that follows, the drawings are not necessarily drawn to scale. As used in the description herein and throughout the claims, the following terms take the meanings explicitly associated herein, unless the context clearly dictates otherwise: the meaning of "a," "an," and "the" includes plural reference, the meaning of "in" includes "in" and "on."

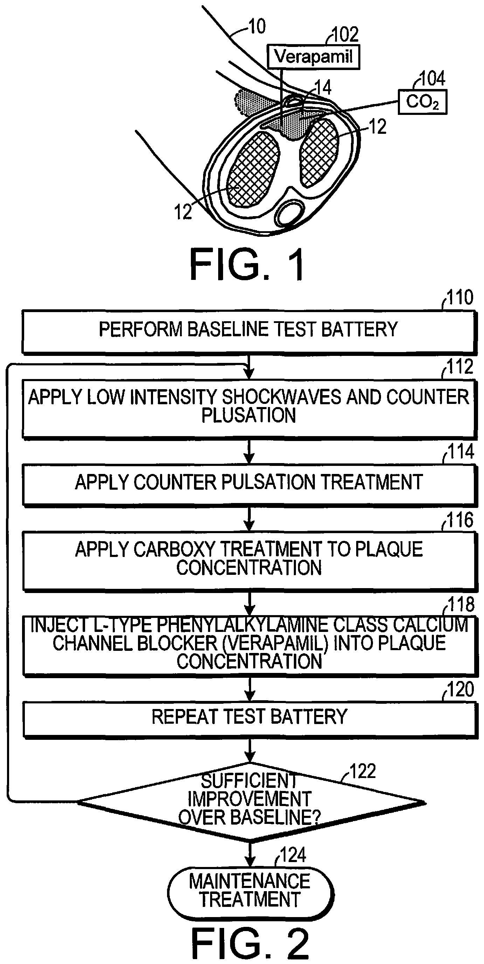

As shown in FIG. 1, Peyronie's disease causes curvature of the penis **10** as the result of a plaque mass **14** forming in the dorsal tunica of the penis **10.** It can also result from calcification of the corpus cavemosa **12** in the penis **10.** As will be shown below, the present treatment protocol includes several steps for improving blood flow through the affected area, along with injecting a therapeutically effective dose of an L-type phenylalkylamine class calcium channel blocker (such as Verapamil) **102** into the plaque mass **14** and also injecting carbon dioxide **104** into the plaque mass **14.**

In one embodiment of a treatment protocol for Peyronie's disease, as shown in FIG. 2, an initial test battery is performed on the patient **110** in order to establish a baseline. This test battery typically includes imaging the plaque with an ultrasound imaging device, measuring blood flow in penile blood vessels of the patient with a duplex Doppler ultrasonography blood flow device and measuring pulse wave velocity in a brachial artery and an ankle artery of the patient. Circulatory blood flow velocity in the patient is tested typically with a duplex Doppler. U.S. Patent No. 6,251,076, issued to Hovland et al., discloses one method of determining blood flow velocity in a penile artery and is referred to for the purpose of disclosing exemplary methods of determining blood flow velocity.

Low intensity shock wave therapy is applied to the plaque mass in the penile region 112. This softens the plaque mass and disrupts calcification in the plaque mass. In doing so, low intensity shockwave (LISW) therapy is applied to the plaque mass for about thirteen treatments over a period of seven to eight weeks, including a two week break period off no low intensity shock wave treatment. In the low intensity shockwave treatment (LISW), shock waves having a maximum energy of 0.09 mJmm2 are applied with a local applicator to the penile area once per day for two or three days per week over a course of five weeks. U.S. Publication No. US-2015/0073312-A1, filed by Ein-Gal, discloses one method of low intensity shockwave treatment and referred to for the purpose of disclosing low intensity shockwave treatment. In a typical treatment, about 300 pulses are applied per minute over the course of between 10 minutes and 20 minutes. The LISW treatment stimulates neovascularization and improves penile blood flow and endothelial function when applied to the corpora cavemosa.

Counter pulsation treatment is applied twice per week during a period of at least ten weeks **114,** which can be done concurrently with the low intensity shock wave therapy. This further disrupts calcification in the plaque mass and improves blood flow. The course of external counter-pulsation treatment includes applying external counter-pulsation treatments to the patient for a predetermined number of days per week for a predetermined number of weeks. In applying the course of external counter-pulsation treatments an electrocardiogram (ECG) sensing device is applied to the patient and the ECG is sensed. U.S. Patent Nos. 7,314,478 and 7,314,478, both issued to Hui, disclose a counter-pulsation apparatus and method for controlling the apparatus and are referred to for the purpose of disclosing exemplary counter-pulsation methods. An inflatable cuff is applied to at least one of the patient's calf, lower thigh, upper thigh or buttocks. Typically, cuffs are applied to both of the lower thighs and to both of the upper thighs. Counter pulsations are applied to the cuffs by inflating the cuffs to a pressure of about 300 mm Hg during a diastole sensed by the ECG. Pressure is then rapidly released from the cuffs during onset of the systole, as sensed by the ECG. Counter-pulsations are performed repeatedly during a treatment sessions that last about one hour, which are performed twice per week over a course of ten weeks. (It should be noted that the term "ECP" is sometimes confused with "EECP," which is a registered a trademark for a brand of ECP. However, the EECP brand can be employed as the type of ECP used.

Carbon dioxide is then injected into the plaque mass **116,** which is referred to as "carboxy therapy." In this step, about 960cc of carbon dioxide is injected into one or both of the corpus cavemosum, the dorsal tunica of the patient or other area of plaque mass (typically in injections of about 160cc each in several different locations). This is typically performed twice per week, but 48 hours apart, for twelve consecutive weeks. Typically, the carboxy therapy is performed after the low intensity shockwave treatment and the counter pulsation treatment steps to reduce the dispersal of the carbon dioxide in the injected tissues. U.S. Patent No. 9,132,245, issued to Mantell, discloses a carboxy therapy application and is referred to disclose one exemplary device and method for administering carboxy therapy. The carboxy therapy infuses carbon dioxide into the tissues, causing the body to interpret the presence of the carbon dioxide as an oxygen deficiency, which results in the production of vascular endothelial growth factors in the tissues. This encourages vascular growth and local reduction in fat tissue, which results in increased blood flow to the corpora cavemosa.

An L-type phenylalkylamine class calcium channel blocker, of the type known generically as "Verapamil," is injected into a calcified plaque area of the penile area **118.** Typically, in this step 0.625 mg to 2.5 mg ofVerapamil is injected into a dorsal tunica of the patient. About 12 Verapamil treatments are administered at a frequency of one every 14 days.

The test battery is repeated 120 to quantify patient treatment progress. If there has not been sufficient improvement over the baseline test, then the treatment steps are repeated **122.** Indicia of sufficient improvement include the observance of no plaque in the ultrasound imaging and the observance of a doubling in blood flow in the affected area. Once the desired result is achieved, the patient can return periodically for examination and maintenance treatments **124** if such treatments are indicated.

The methods of the present invention could also be useful in treating calcification in an individual's hand, or other extremity.

The above described embodiments, while including the preferred embodiment and the best mode of the invention known to the inventor at the time of filing, are given as illustrative examples only. It will be readily appreciated that many deviations may be made from the specific embodiments disclosed in this specification without departing from the spirit of the invention. Accordingly, the scope of the invention is to be determined by the claims below rather than being limited to the specifically described embodiments above.

## Claims

1. Carbon dioxide for use in a method of treating Peyronie's disease in a patient having diffused plaque and a plaque mass associated with Peyronie's disease in a penile region, the method comprising the steps of:
(a) performing a battery of tests to quantify an initial state of parameters associated with Peyronie's disease in the patient;
(b) applying low intensity shock wave therapy to the plaque mass in the penile region, thereby softening the plaque mass and disrupting any calcification in the plaque mass;
(c) injecting the carbon dioxide into the plaque mass after the step of applying low intensity shock wave therapy;
(d) repeating the battery of tests to quantify a current state of parameters associated with Peyronie's disease in the patient and comparing the current state to the initial state; and
(e) until the current state differs from the initial state by at least a predetermined amount, repeating steps (b) through (d).

2. Carbon dioxide for use according to claim 1, wherein the method further comprises the step of applying external counter pulsation therapy to the patient concurrently with the step of applying low intensity shock wave therapy, further softening the plaque mass and disrupting any calcification in the plaque mass.

3. Carbon dioxide for use according to claim 2, wherein the step of applying counter-pulsation therapy comprises applying a counter pulsation treatment about twice per week during a period of at least ten weeks.

4. Carbon dioxide for use according to any one of claims 1 to 3, wherein the step of applying low intensity shock wave therapy to the plaque mass comprises applying low intensity shock wave therapy to the plaque mass for about thirteen treatments over a period of seven to eight weeks, including a two week break period off no low intensity shock wave treatment.

5. Carbon dioxide for use according to any one of claims 1 to 4, wherein the method further comprises the step of injecting a therapeutically effective dose of an L-type phenylalkylamine class calcium channel blocker, preferably Verapamil, into a calcified plaque area of the penile area.

6. Carbon dioxide for use according to claim 5, wherein the step of injecting a therapeutically effective dose of an L-type phenylalkylamine class calcium channel blocker, preferably Verapamil, is performed after the step of applying low intensity shock wave therapy to the plaque mass.

7. Carbon dioxide for use according to claim 5 or 6, wherein the step of injecting a therapeutically effective dose of an L-type phenylalkylamine class calcium channel blocker, preferably Verapamil, comprises the step of injecting between 0.625 mg to 2.5 mg into a dorsal tunica of the patient.

8. Carbon dioxide for use according to any one of claims 5 to 7, wherein the step of injecting a therapeutically effective dose of an L-type phenylalkylamine class calcium channel blocker, preferably Verapamil, comprises performing about 12 treatments at a frequency of every 14 days.

9. Carbon dioxide for use according to any one of claims 1 to 8, wherein the step of injecting carbon dioxide into the plaque mass comprises injecting
about 960 cc (cm³) of carbon dioxide into a dorsal tunica or corpus cavernosum or other area of plaque mass of the patient; and/or
a plurality of doses of about 160 cc (cm³) of carbon dioxide per dose into the plaque mass for a total of 960 cc after the steps pf applying low intensity shock wave therapy and optionally applying a counter pulsation treatment.

10. Carbon dioxide for use according to any one of claims 1 to 9, wherein the step of injecting carbon dioxide into the plaque mass is performed twice per week, but 48 hours apart, for twelve consecutive weeks.

11. Carbon dioxide for use according to any one of claims 1 to 10, wherein the battery of tests includes
imaging the plaque with an ultrasound imaging device and the current state differs from the initial state by the predetermined amount when no plaque is observed; and/or
measuring blood flow in penile blood vessels of the patient with a duplex Doppler ultrasonography blood flow device and the current state differs from the initial state by the predetermined amount when about a doubling in blood flow is observed; and/or
measuring pulse wave velocity in a brachial artery and an ankle artery of the patient.

## Patentansprüche

1. Kohlendioxid zur Verwendung in einem Verfahren zum Behandeln der Peyronie-Krankheit bei einem Patienten mit diffusem Plaque und einer mit der Peyronie-Krankheit assoziierten Plaquemasse in einer Penisregion, wobei das Verfahren die Schritte umfasst:
(a) Durchführen einer Reihe von Tests, um einen Anfangszustand von Parametern, die mit der Peyronie-Krankheit bei dem Patienten assoziiert sind, zu quantifizieren;
(b) Anwenden einer Schockwellentherapie mit geringer Intensität an der Plaquemasse in der Penisregion, wodurch die Plaquemasse erweicht und jegliche Verkalkung in der Plaquemasse zerstört wird;
(c) Injizieren des Kohlendioxids in die Plaquemasse nach dem Schritt des Anwendens der Schockwellentherapie mit geringer Intensität;
(d) Wiederholen der Reihe von Tests, um einen aktuellen Zustand von Parametern, die mit der Peyronie-Krankheit bei dem Patienten assoziiert sind, zu quantifizieren und Vergleichen des aktuellen Zustands mit dem Anfangszustand; und
(e) Wiederholen der Schritte (b) bis (d), bis sich der aktuelle Zustand von dem Anfangszustand um mindestens einen vorbestimmten Betrag unterscheiden.

2. Kohlendioxid zur Verwendung gemäß Anspruch 1, wobei das Verfahren ferner den Schritt des Anwendens einer externen Gegenpulsationstherapie bei dem Patienten gleichzeitig mit dem Schritt des Anwendens der Schockwellentherapie mit geringer Intensität umfasst, ferner das Erweichen der Plaquemasse und das Zerstören jeglicher Verkalkung in der Plaquemasse.

3. Kohlendioxid zur Verwendung gemäß Anspruch 2, wobei der Schritt des Anwendens der Gegenpulsationstherapie das Anwenden einer Gegenpulsationsbehandlung etwa zweimal pro Woche über einen Zeitraum von mindestens 10 Wochen umfasst.

4. Kohlendioxid zur Verwendung gemäß einem der Ansprüche 1 bis 3, wobei der Schritt des Anwendens der Schockwellentherapie mit geringer Intensität auf die Plaquemasse das Anwenden der Schockwellentherapie mit geringer Intensität auf die Plaquemasse für ungefähr 13 Behandlungen über einen Zeitraum von sieben bis acht Wochen umfasst, einschließlich einer zweiwöchigen Pause ohne Schockwellenbehandlung mit geringer Intensität.

5. Kohlendioxid zur Verwendung gemäß einem der Ansprüche 1 bis 4, wobei das Verfahren ferner den Schritt des Injizierens einer therapeutisch-wirksamen Dosis eines Calciumkanalblockers der Phenylalkylamin-Klasse vom L-Typ, bevorzugt Verapamil, in einen verkalkten Plaquebereich des Penisbereichs umfasst.

6. Kohlendioxid zur Verwendung gemäß Anspruch 5, wobei der Schritt des Injizierens einer therapeutisch-wirksamen Dosis eines Calciumkanalblockers der Phenylalkylamin-Klasse vom L-Typ, bevorzugt Verapamil, nach dem Schritt des Anwendens der Schockwellentherapie mit geringer Intensität auf die Plaquemasse durchgeführt wird.

7. Kohlendioxid zur Verwendung gemäß Anspruch 5 oder 6, wobei der Schritt des Injizierens einer therapeutisch-wirksamen Dosis eines Calciumkanalblockers der Phenylalkylamin-Klasse vom L-Typ, bevorzugt Verapamil, den Schritt des Injizierens zwischen 0,625 mg bis 2,5 mg in eine dorsale Tunica des Patienten umfasst.

8. Kohlendioxid zur Verwendung gemäß einem der Ansprüche 5 bis 7, wobei der Schritt des Injizierens einer therapeutisch-wirksamen Dosis eines Calciumkanalblockers der Phenylalkylamin-Klasse vom L-Typ, bevorzugt Verapamil, das Durchführen von etwa 12 Behandlungen mit einer Häufigkeit von alle 14 Tagen umfasst.

9. Kohlendioxid zur Verwendung gemäß einem der Ansprüche 1 bis 8, wobei der Schritt des Injizierens von Kohlendioxid in die Plaquemasse das Injizieren
von etwa 960 cc (cm³) Kohlendioxid in eine dorsale Tunica oder den Corpus cavernosum oder einen anderen Bereich der Plaquemasse des Patienten umfasst; und/oder
einer Vielzahl von Dosen von etwa 160 cc (cm³) Kohlendioxid pro Dosis in die Plaquemasse für insgesamt 960 cc nach den Schritten des Anwendens der Schockwellentherapie mit geringer Intensität und gegebenenfalls des Anwendens einer Gegenpulsationsbehandlung umfasst.

10. Kohlendioxid zur Verwendung gemäß einem der Ansprüche 1 bis 9, wobei der Schritt des Injizierens von Kohlendioxid in die Plaquemasse zweimal pro Woche, aber im Abstand von 48 Stunden, für zwölf aufeinanderfolgende Wochen durchgeführt wird.

11. Kohlendioxid zur Verwendung gemäß einem der Ansprüche 1 bis 10, wobei die Reihe der Tests umfasst
Abbilden des Plaques mit einer Ultraschall-Bildgebungsvorrichtung und wobei sich der aktuelle Zustand vom Anfangszustand um den vorbestimmten Betrag, wenn kein Plaque beobachtet wird, unterscheidet; und/oder
Messen des Blutflusses in Penisblutgefäßen des Patienten mit einer Duplex-Doppler-Ultraschall-Blutflussvorrichtung, und wobei sich der aktuelle Zustand vom Anfangszustand um den vorbestimmten Betrag, wenn etwa eine Verdoppelung des Blutflusses beobachtet wird, unterscheidet; und/oder
Messen der Pulswellengeschwindigkeit in einer Brachialarterie und einer Knöchelarterie des Patienten.

## Revendications

1. Dioxyde de carbone pour une utilisation dans une méthode de traitement de la maladie de La Peyronie chez un patient ayant une plaque diffuse et une masse de plaque associées à la maladie de La Peyronie dans une région pénienne, la méthode comprenant les étapes suivantes :
(a) réalisation d'une batterie de tests pour quantifier un état initial de paramètres associés à la maladie de La Peyronie chez le patient ;
(b) application d'une thérapie par ondes de choc de faible intensité à la masse de plaque dans la région pénienne, ce qui assouplit ainsi la masse de plaque et rompt toute calcification dans la masse de plaque ;
(c) injection du dioxyde de carbone dans la masse de plaque après l'étape d'application d'une thérapie par ondes de choc de faible intensité ;
(d) répétition de la batterie de tests pour quantifier un état actuel de paramètres associés à la maladie de La Peyronie chez le patient et comparer l'état actuel à l'état initial ; et
(e) à moins que l'état actuel diffère de l'état initial au moins d'une quantité prédéterminée, répétition des étapes (b) à (d).

2. Dioxyde de carbone pour une utilisation selon la revendication 1, dans lequel la méthode comprend en outre l'étape d'application d'une thérapie par contre-impulsions externe au patient en même temps que l'étape d'application d'une thérapie par ondes de choc de faible intensité, ce qui assouplit encore la masse de plaque et rompt toute calcification dans la masse de plaque.

3. Dioxyde de carbone pour une utilisation selon la revendication 2, dans lequel l'étape d'application d'une thérapie par contre-impulsions comprend l'application d'un traitement par contre-impulsions environ deux fois par semaine sur une période d'au moins dix semaines.

4. Dioxyde de carbone pour une utilisation selon l'une quelconque des revendications 1 à 3, dans lequel l'étape d'application d'une thérapie par ondes de choc de faible intensité à la masse de plaque comprend l'application d'une thérapie par ondes de choc de faible intensité à la masse de plaque pendant environ treize traitements sur une période de sept à huit semaines, comprenant une période d'arrêt de deux semaines sans traitement par ondes de choc de faible intensité.

5. Dioxyde de carbone pour une utilisation selon l'une quelconque des revendications 1 à 4, dans lequel la méthode comprend en outre l'étape d'injection d'une dose efficace du point de vue thérapeutique d'un inhibiteur calcique de la classe des phénylalkylamines de classe L, de préférence de vérapamil, dans une zone de plaque calcifiée de la zone pénienne.

6. Dioxyde de carbone pour une utilisation selon la revendication 5, dans lequel l'étape d'injection d'une dose efficace du point de vue thérapeutique d'un inhibiteur calcique de la classe des phénylalkylamines de classe L, de préférence de vérapamil, est effectuée après l'étape d'application d'une thérapie par ondes de choc de faible intensité à la masse de plaque.

7. Dioxyde de carbone pour une utilisation selon la revendication 5 ou 6, dans lequel l'étape d'injection d'une dose efficace du point de vue thérapeutique d'un inhibiteur calcique de la classe des phénylalkylamines de classe L, de préférence de vérapamil, comprend l'étape d'injection d'entre 0,625 mg et 2,5 mg dans la tunique dorsale du patient.

8. Dioxyde de carbone pour une utilisation selon l'une quelconque des revendications 5 à 7, dans lequel l'étape d'injection d'une dose efficace du point de vue thérapeutique d'un inhibiteur calcique de la classe des phénylalkylamines de classe L, de préférence de vérapamil, comprend la réalisation d'environ 12 traitements à une fréquence d'un tous les 14 jours.

9. Dioxyde de carbone pour une utilisation selon l'une quelconque des revendications 1 à 8, dans lequel l'étape d'injection de dioxyde de carbone dans la masse de plaque comprend l'injection
d'environ 960 cm³ de dioxyde de carbone dans la tunique dorsale ou le corps caverneux ou une autre zone de masse de plaque du patient ; et/ou
de plusieurs doses d'environ 160 cm³ de dioxyde de carbone par dose dans la masse de plaque pour un total de 960 cm³ après les étapes d'application d'une thérapie par ondes de choc de faible intensité et éventuellement d'application d'un traitement par contre-impulsions.

10. Dioxyde de carbone pour une utilisation selon l'une quelconque des revendications 1 à 9, dans lequel l'étape d'injection de dioxyde de carbone dans la masse de plaque est effectuée deux fois par semaine, mais avec un espacement de 48 heures, pendant douze semaines consécutives.

11. Dioxyde de carbone pour une utilisation selon l'une quelconque des revendications 1 à 10, dans lequel la batterie de tests comprend
l'imagerie de la plaque avec un dispositif d'imagerie par ultrasons, l'état actuel différant de l'état initial de la quantité prédéterminée quand aucune plaque n'est observée ; et/ou
la mesure du débit sanguin dans les vaisseaux sanguins péniens du patient avec un dispositif de mesure de débit sanguin par ultrasonographie Doppler en duplex, l'état actuel différant de l'état initial de la quantité prédéterminée quand environ un doublement du débit sanguin est observé ; et/ou
la mesure de la vitesse d'une onde pulsée dans une artère brachiale et une artère de cheville du patient.
